# EUROPÄISCHE PATENTANMELDUNG

(11) **EP 2 325 288 A1**
(43) Veröffentlichungstag der Anmeldung: **25.05.2011**
(21) Anmeldenummer: 09176684.0
(22) Anmeldetag: 20.11.2009
(51) Int. Cl.: C10J 3/66, C07C 29/151, C10G 2/00

(54) **Verfahren und Anlage zur thermisch-chemischen Verarbeitung und Verwertung von kohlenstoffhaltigen Substanzen**

(71) Anmelder: RV Lizenz AG, 6300 Zug (CH)
(72) Erfinder: Rüdlinger, Mike, 8404 Winterthur (CH)
(74) Vertreter: Rentsch & Partner

(57) **Zusammenfassung**

Bei einem Verfahren zur thermisch-chemischen Verwertung von kohlenstoffhaltigen Substanzen (A1), werden in einer ersten Stufe (11) die kohlenstoffhaltigen Substanzen (A1) zugeführt und pyrolysiert, wobei Pyrolysekoks (D) und Pyrolysegas (E) entstehen. In einer zweiten Stufe (12) wird der Pyrolysekoks (D) aus der ersten Stufe (11) vergast, wobei Synthesegas (S) entsteht, und Schlacke und andere Reststoffe (H, K) übrig bleiben und abgeführt werden. In einer dritten Stufe (13) wird das Synthesegas (S) aus der zweiten Stufe (12) in Kohlenwasserstoffe und/oder andere Produkte (Q) umgewandelt, die abgeführt werden. Überschüssiges Rücklaufgas (R) aus der dritten Stufe (13) wird in die erste Stufe (11) und/oder die zweite Stufe (12) geleitet, wobei die drei Stufen (11, 12, 13) einen geschlossenen Kreislauf bilden.

## Beschreibung

### Technisches Gebiet

Die Erfindung betrifft Verfahren und Anlagen zur emissionsfreien thermisch-chemischen Verarbeitung und Verwertung von festen, flüssigen und gasförmigen kohlenstoffhaltigen Substanzen und Gemischen, insbesondere von Kohle, Biomasse und anderen heterogenen Materialien.

### Stand der Technik

Es sind aus dem Stand der Technik verschiedene Typen von Verfahren und Anlagen bekannt, mit denen aus festen, flüssigen und gasförmigen kohlenstoffhaltigen Substanzen Gasgemische hergestellt werden können, die anschliessend als sogenanntes Synthesegas für chemische Synthesen verwendet werden. Synthesegase mit Kohlenmonoxid und Wasserstoff werden beispielsweise für die industrielle Liquid-Phase-Methanol-Synthese oder die für Fischer-Tropsch-Synthese zur Produktion von Kohlenwasserstoffen und anderen organischen Substanzen verwendet. Alternativ können solche Synthesegase auch zur Erzeugung von Energie verwendet werden, beispielsweise als Brennstoff zum Betrieb von Wärmekraftmaschinen.

Zur Herstellung von Kohlenmonoxid-Wasserstoff-Synthesegasen aus festem Kohlenstoff wird dieser mit Hilfe von Sauerstoff, Kohlendioxid oder Wasser zu Synthesegas vergast:

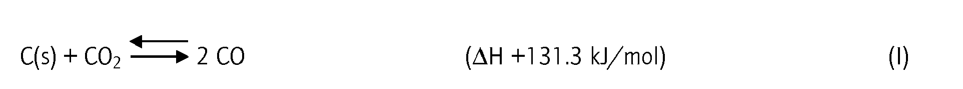

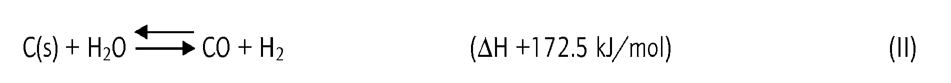

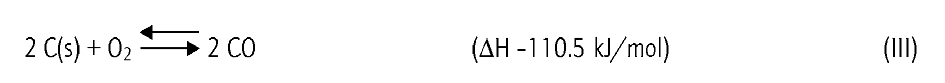

Das Verhältnis zwischen Kohlenmonoxid und Wasserstoff ist durch die sogenannte Wassergas-Shift-Reaktion IV gegeben:

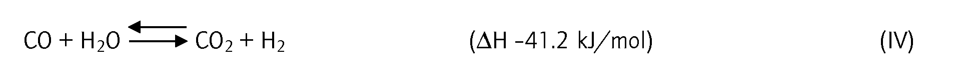

Die für den Ablauf der endothermen Reaktionen I und II notwendige thermische Energie kann beispielsweise aus einer partielle Verbrennung des festen Kohlenstoffs in der Reaktion III stammen, oder extern zugeführt werden.

Bei einem bekannten Verfahrenstyp zur Herstellung von Synthesegas bzw. entsprechendem gasförmigen Brennstoff liegt der feste Kohlenstoff für die Vergasungsreaktionen in Form von Koks vor. Dieser wird wiederum in einer vorangehenden Stufe durch Pyrolyse von Kohle oder anderen kohlenstoffhaltigen Substanzen erzeugt. Die bei der Pyrolyse entstehenden Pyrolysegase werden verbrannt, wobei die heissen kohlendioxidhaltigen Verbrennungsgase zum einen als Vergasungsmittel für den Koks als auch als externer Wärmeenergielieferant dienen.

Bei einem anderen Verfahrenstyp wird der Koks unter Zugabe von Luft/Sauerstoff vergast, wobei die thermische Energie primär durch die partielle Verbrennung des Kohlenstoffs des Kokses erfolgt. Pyrolysegas aus einer vorangehenden Pyrolysestufe wird anschliessend in das heisse Synthesegas gemischt, wo es gecrackt wird, so dass ein teerfreies brennbares Gasgemisch entsteht.

Die bekannten Verfahren zur Herstellung von Synthesegas sind darauf ausgerichtet, aus kostengünstiger fossiler Kohle Synthesegas für die chemische Industrie herzustellen, beispielsweise zur Herstellung von flüssigem Treibstoff und anderen hochwertigen Endprodukten. In diesen Verfahren wird ein Teil des Ausgangsmaterials zur Energiegewinnung verbrannt, so dass bei Herstellung der hochwertigen Endprodukte grosse, nicht weiter nutzbare Kohlendioxidmengen anfallen. Aufgrund der menschenverursachten Klimaerwärmung sind solche ineffizienten Verfahren heute immer weniger akzeptabel.

Andere solche Verfahren sind primär darauf ausgerichtet, aus festen kohlenstoffhaltigen Substanzen wie beispielsweise fossiler Kohle, Biomasse, oder heterogenen Gemischen wie beispielsweise brennbarem Abfall leichter handhabbaren gasförmigen Brennstoff herzustellen. Mit diesem können dann zum Beispiel Gasturbinen betrieben werden, welche einen hohen Wirkungsgrad aufweisen. Jedoch wird auch bei diesen Verfahren ein Teil der im festen Ausgangmaterial gespeicherten chemischen Energie bei der Umwandlung, entweder bei der Koksherstellung oder bei der Gasherstellung, verbraucht und entsprechend Kohlendioxid freigesetzt, was die Kohlendioxideffizienz senkt.

Ein Nachteil der bekannten Verfahren ist neben der geringen Effizienz der komplizierte Aufbau, insbesondere bei Anlagen, bei welchem Koks im Wirbelstrom oder Flugstrom vergast wird. Solche Verfahren benötigen zudem eine aufwendige Steuerung.

### Aufgabe der Erfindung

Aufgabe der Erfindung ist es, Verfahren und Anlagen zur thermisch-chemischen Verarbeitung und Verwertung von festen, flüssigen und gasförmigen kohlenstoffhaltigen Substanzen und Gemischen, insbesondere von Kohle, Biomasse und anderen heterogenen Materialien zur Verfügung zu stellen, welche die oben erwähnten und andere Nachteile nicht aufweisen.

Erfindungsgemässe Verfahren und Anlagen sollen möglichst emissionsfrei sein und eine minimale Kohlendioxidproduktion aufweisen.

Eine andere Aufgabe der Erfindung ist es, Verfahren und Anlagen zur Verfügung zu stellen, mit welchen Biomasse mit möglichst wenig externer Energiezufuhr in andere energiereiche Produkte umgewandelt werden kann.

Noch eine Aufgabe der Erfindung ist es, Verfahren und Anlagen zur Verfügung zu stellen, mit denen schwierig zu verwertende erdölhaltige Substanzen wie beispielsweise Ölschiefer, Ölsand oder Ölschlamm emissionsfrei in Produkte umgewandelt werden können.

Eine weitere Aufgabe der Erfindung ist es, Verfahren und Anlagen zur Verfügung zu stellen, mit welchen feste, flüssige oder gasförmige Substanzen effizient in gasförmige oder flüssige Energieträger umgewandelt werden können.

Noch eine Aufgabe der Erfindung ist es, Verfahren und Anlagen zur Verfügung zu stellen, mit welchen flüssige und gasförmige Brennstoffe und Treibstoffe mit einer möglichst neutralen Kohlendioxidbilanz erzeugt werden können.

Diese und andere Aufgaben werden gelöst durch erfindungsgemässe Verfahren und erfindungsgemässe Anlagen gemäss den unabhängigen Ansprüchen. Weitere bevorzugte Ausführungsformen sind in den abhängigen Ansprüchen gegeben.

### Darstellung der Erfindung

Bei einem Verfahren zur thermisch-chemischen Verwertung von kohlenstoffhaltigen Substanzen werden in einer ersten Stufe die kohlenstoffhaltigen Substanzen zugeführt und pyrolysiert, wobei Pyrolysekoks und Pyrolysegas entstehen. In einer zweiten Stufe wird der Pyrolysekoks aus der ersten Stufe vergast, wobei Synthesegas entsteht, und Schlacke und andere Reststoffe übrig bleiben und abgeführt werden. In einer dritten Stufe wird das Synthesegas aus der zweiten Stufe in Kohlenwasserstoffe und/oder andere Produkte umgewandelt, die abgeführt werden. Überschüssiges Rücklaufgas aus der dritten Stufe wird in die erste Stufe und/oder die zweite Stufe geleitet. Die drei Stufen bilden einen geschlossenen Kreislauf.

Das Verfahren kann in allen drei Stufen unter Druck durchgeführt werden. Das Pyrolysegas aus der ersten Stufe kann in die zweite Stufe und/oder in die dritte Stufe geleitet werden. Das Synthesegas aus der zweiten Stufe wiederum kann in die dritte Stufe und/oder die erste Stufe geleitet werden.

Vorteilhaft verläuft der Gasstrom innerhalb des Kreislaufs in einer bestimmten Richtung. Der Gasstrom kann beispielsweise innerhalb des Kreislaufs von der ersten Stufe über die zweite

Stufe zur dritten Stufe und wieder zur ersten Stufe fliessen, oder von der ersten Stufe über die dritte Stufe zur zweiten Stufe und wieder zurück zur ersten Stufe.

Besonders vorteilhaft liegt entlang des Kreislaufs ein Druckgefälle vor. Dies erlaubt die Förderung des Gasstroms entlang des Kreislaufs ohne zusätzliches Fördersystem, mit der Ausnahme eines Verdichters zur Erzeugung des Druckgefälles.

Die erste Stufe des Verfahrens kann in einem oder mehreren Druckreaktoren durchgeführt werden.

Die Zufuhr der Wärmeenergie für die Pyrolysereaktionen in der ersten Stufe kann zum Teil oder vollständig durch Rückleiten eines Teils des heissen Synthesegases aus der zweiten Stufe in die erste Stufe erfolgen, und/oder durch partielle Oxidation des Ausgangsmaterials und des entstehenden Pyrolysekokses.

Vorteilhaft wird die erste Stufe bei einer Temperatur zwischen 300 und 800 °C, bevorzugt zwischen 450 und 700 °C, und besonders bevorzugt zwischen 500 und 600 °C, durchgeführt.

Die zweite Stufe des Verfahrens kann ebenfalls in einem oder mehreren zweiten Druckreaktoren durchgeführt werden. Für die Vergasungsreaktion in der zweiten Stufe kann Sauerstoff und/oder Wasserdampf und/oder Kohlendioxid als Vergasungsmittel verwendet werden.

Der Pyrolysekoks kann vollständig oder nur teilweise vergast werden. Im letzteren Fall kann der nicht prozessierte Koks zusammen mit der anfallenden Schlacke deponiert werden.

Die in der zweiten Stufe die für die Vergasungsreaktion notwendige thermische Energie kann zum Teil oder vollständig von aussen zugeführt werden, beispielsweise durch Heizvorrichtungen und/oder Wärmetauscher, und/oder durch Oxidation eines Teils des Pyrolysekokses mit einem Oxidationsmittel, insbesondere Sauerstoff, erzeugt werden.

Vorteilhaft wird die zweite Stufe des erfindungsgemässen Verfahrens bei einer Temperatur zwischen 600 und 1600 °C, bevorzugt zwischen 700 und 1400 °C, und besonders bevorzugt zwischen 850 und 1000 °C, durchgeführt.

Die Temperatur in der zweiten Stufe beträgt in einer bevorzugten Variante 850 °C oder mehr, wobei der Pyrolysekoks und die Pyrolysegase während mindestens 2 Sekunden in der zweiten Stufe verbleiben. Auf diese Art und Weise werden die in vielen Ländern geltenden Vorschriften zur Behandlung schadstoffkontaminierter Materialien und Sonderabfälle erfüllt.

Vorteilhaft wird die erste Stufe und/oder die zweite Stufe des erfindungsgemässen Verfahrens bei einem Druck zwischen 10 und 60 bar, vorzugweise zwischen 10 und 25 bar, durchgeführt.

In einer anderen Variante des Verfahrens wird die erste Stufe und die zweite Stufe im gleichen Druckreaktor durchgeführt.

Die dritte Stufe des Verfahrens wird vorteilhaft in einem oder mehreren Druckreaktoren durchgeführt. Die Umwandlung in der dritten Stufe erfolgt bevorzugt mit einer Fischer-Tropsch-Synthese oder einer Liquid-Phase-Methanol-Synthese.

Das erfindungsgemässe Verfahren wird vorteilhaft mit einer nachfolgend beschriebenen erfindungsgemässen Anlage durchgeführt wird.

Eine solche erfindungsgemässe Anlage zur thermisch-chemischen Verwertung von kohlenstoffhaltigen Substanzen beinhaltet einen ersten Anlagenteil zur Durchführung einer Pyrolyse der kohlenstoffhaltigen Substanzen zu Pyrolysekoks und Pyrolysegas, einen zweiten Anlagenteil zur Durchführung einer Vergasung des Pyrolysekokses zu Synthesegas und Reststoffen; und einen dritten Anlagenteil zur Durchführung einer Umwandlung des Synthesegases in Kohlenwasserstoffe und/oder andere Produkte. Alle drei Anlageteile sind druckfest geschlossen und bilden einen im Wesentlichen geschlossenen Kreislauf. Eine Transportleitung für das Pyrolysegas verbindet den ersten Anlagenteil druckfest mit dem zweiten Anlagenteil und/oder mit dem dritten Anlagenteil. Eine Transportleitung für das Synthesegas verbindet den zweiten Anlagenteil druckfest mit dem dritten Anlagenteil und/oder mit dem ersten Anlagenteil. Eine Transportleitung für das Rücklaufgas wiederum verbindet den dritten Anlagenteil druckfest mit dem ersten Anlagenteil und/oder mit dem zweiten Anlagenteil.

Vorteilhaft ist entlang mindestens einer der genannten Transportleitungen mindestens ein Verdichter angeordnet ist.

Es können Mittel vorgesehen sein, welche einen Gasstrom entlang der Transportleitungen nur in einer bestimmten Richtung fliessen lassen, vorzugsweise vom ersten Anlagenteil über den zweiten Anlagenteil zum dritten Anlagenteil und wieder zum ersten Anlagenteil, oder vom ersten Anlagenteil über den dritten Anlagenteil zum zweiten Anlagenteil und wieder zum ersten Anlagenteil.

Die Anlagenteile können je einen oder mehrere Druckreaktoren aufweisen. In einer vorteilhaften Variante weisen der erste und/oder der zweite Anlagenteil Heizvorrichtungen und/oder Wärmetauscher auf.

Es kann eine Verzweigung der Transportleitung des Synthesegases vorgesehen sein, mit welcher aus dem zweiten Anlagenteil ein Teil des Synthesegases in den ersten Druckreaktor zurück geleitet werden kann.

In einer anderen vorteilhaften Variante einer erfindungsgemässen Anlage weist der erste Anlagenteil und der zweite Anlagenteil einen gemeinsamen Druckreaktor auf.

Der dritte Anlagenteil umfasst vorzugsweise eine Fischer-Tropsch-Synthese-Anlage oder einer Liquid-Phase-Methanol-Synthese-Anlage.

Eine erfindungsgemässe Anlage ist bevorzugt dazu eingereichtet ein erfindungsgemässes Verfahren durchzuführen.

Besonders vorteilhaft ist eine Anlage, die derart gefahren werden kann, dass von der ersten Stufe über die zweite Stufe zur dritten Stufe ein Druckgefälle vorliegt. Auf diese Weise erfolgt der Massentransport entlang dem zyklischen Gasstrom getrieben vom Druckunterschied zwischen den verschiedenen Druckreaktoren.

Erfindungsgemässe Kohlenwasserstoffe und andere Produkte werden hergestellt mit einem erfindungsgemässen Verfahren, beziehungsweise mit einer erfindungsgemässen Anlage. Sie können von analogen Erdölprodukten beispielsweise durch das Fehlen typischer Schwefel-und Phosphor-Verunreinigungen unterschieden werden, oder bei einer Herstellung mit Anteilen des Ausgangsmaterials aus Biomasse durch erhöhte C14-Isotop-Anteile.

### Ausführung der Erfindung

Zum besseren Verständnis der vorliegenden Erfindung wird nachfolgend auf die Zeichnungen Bezug genommen, die verschiedene mögliche Ausführungsform einer erfindungsgemässen Anlage zu Durchführung des erfindungsgemässen Verfahrens.

Die im Folgenden gegebenen Beispiele werden zur besseren Veranschaulichung gegeben, sind jedoch nicht dazu geeignet, die Erfindung auf die hierin offenbarten Merkmale zu beschränken.

### Erstes Ausführungsbeispiel

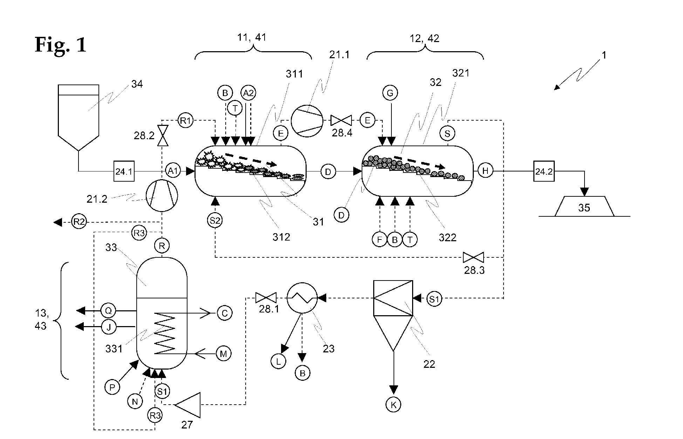

Eine erste Variante einer erfindungsgemässen Anlage zur thermisch-chemischen Verwertung von kohlenstoffhaltigen Feststoffen mit einem erfindungsgemässen Verfahren ist in Figur 1 schematisch dargestellt. Die Anlage 1 enthält drei Anlagenteile 41, 42, 43 zur Durchführung der drei Stufen 11, 12, 13 des erfindungsgemässen Verfahrens, die so zu einem geschlossenen Kreislauf verbunden sind, dass sie einen zyklischen Gasstrom erlauben. Im ersten Anlagenteil 41 (Pyrolyse-Stufe 11) wird kohlenstoffhaltiges Ausgangsmaterial unter Druck pyrolysiert, wodurch Pyrolysekoks und Pyrolysegase entstehen. In einem zweiten Anlagenteil 42 (Vergasungs-Stufe 12) wird Pyrolysekoks zu Synthesegas vergast, welches schliesslich in einem dritten Anlagenteil 43 (Synthese-Stufe 13) zu Kohlenwasserstoffen oder anderen Produkten reagiert wird. Die Edukte - die zu verarbeitenden kohlenstoffhaltigen Ausgangsmaterialien A1 - werden laufend über den Anlageteil 41 in den Kreislauf eingespeist. Gleichzeitig werden die aus dem Synthesegas erzeugten Produkte Q kontinuierlich aus der dritten Stufe abgezogen, sowie die verschiedenen Reststoffe H, K, J laufend aus dem Kreislauf entfernt.

Der erste Anlagenteil umfasst im gezeigten Beispiel einen ersten Druckreaktor 31, in welchem unter Druck eine Pyrolyse des kohlenstoffhaltigen Ausgangsmaterials A1 stattfindet. Das Ausgangsmaterial A1 wird dazu über eine geeignete Druckschleuse 24.1 in den unter Druck stehenden Pyrolysereaktor 31 eingebracht. In der gezeigten Ausgestaltungsform besteht der Pyrolysereaktor 31 aus einem horizontalen Druckkörper 311, in welchem die horizontale Förderung des Materials entlang des Reaktors während der Pyrolyse durch einen schematisch Vorschubrost 312 mit hin und her bewegten Rostplatten erfolgt. Jede andere für den kontinuierlichen Vorschub des zu verarbeitenden Ausgangmaterials geeignete Förderungsvorrichtung ist ebenfalls anwendbar, beispielsweise Walzenroste, Kettenförderer, Förderschnecken, etc., sind ebenfalls einsetzbar. Auch ein Drehrohrofen kann eingesetzt werden.

Als Ausgangsmaterial für ein erfindungsgemässes Verfahren kann eine Vielzahl kohlenstoffhaltiger Substanzen dienen, insbesondere Kohle, Biomasse, Abfälle sowie andere heterogene Substanzen wie beispielsweise verunreinigtes Erdreich. Auch schwierig zu verwertende festflüssige erdölhaltige Substanzen wie Ölschiefer, Ölsand, oder Ölschlamm können in einem erfindungsgemässen Verfahren verwertet werden. Als Zuschlagstoff können auch gasförmige kohlenstoffhaltige Nebenprodukte der Erdölindustrie verwendet werden, die ansonsten nicht weiter verwertet werden können und ansonsten abgefackelt werden müssten.

Der Brennwert der Ausgangsmaterialien, der Kohlenstoffgehalt, Wassergehalt, sowie der Gehalt an nicht brennbaren Reststoffen wie Metall, Glas und Keramik, kann stark variieren. Das Ausgangsmaterial kann auf eine für eine bestimmte Anlage am besten geeignete Stückgrösse verkleinert werden, wobei sich die bevorzugte Stückgrösse aus der Konsistenz des Materials und aus der konkreten Ausgestaltung des ersten Druckreaktors bzw. des reaktorinternen Fördersystems ergibt. Zur Verarbeitung mit einem Vorschubrost ist beispielsweise eine Stückgrösse von ca. 5-10 cm gut geeignet.

Im Pyrolysereaktor 31 wird das Material bei einer Temperatur von ca. 300-800°C und einem Druck von 10 bis 60 bar kontinuierlich durch den Druckreaktor 31 transportiert und dabei unter Sauerstoffausschluss pyrolysiert. Die Temperatur wird dabei unter anderem so gewählt, dass neben der Aufrechterhaltung der Pyrolysereaktion der gewünschte Betriebsdruck gehalten wird, zum einen aufgrund der Ausdehnung der Gase aufgrund der Temperatur, und zum anderen aufgrund der Neuproduktion von Pyrolysegasen. Eine Minimaltemperatur von 450 °C stellt eine stetige Abreaktion von freien Sauerstoffverbindungen während der Pyrolyse sicher. Besonders gut geeignet sind eine Betriebstemperatur von 500-600 °C und ein Betriebsdruck zwischen 10 und 25 bar. Die für die Pyrolysereaktionen notwendige thermische Energie stammt zum einen durch aus dem heissen Feedback-Gasstrom S2 aus dem zweiten Reaktor 32, auf den nachfolgend noch eingegangen wird, und die Zugabe von Prozessdampf B der Aufrechterhaltung der Betriebstemperatur des ersten Reaktors. Ebenfalls vorhanden sein kann eine externe Wärmezufuhr wie beispielsweise ein Wärmetauscher oder eine externe Heizung. Letztere ist auch vorteilhaft bei der Inbetriebnahme einer erfindungsgemässen Anlage 1 aus dem Kaltzustand.

Rücklaufgas R aus der dritten Stufe 13 wird nach dem Passieren eines Verdichters 21.2 dem ersten Druckreaktor 31 zugeführt. Das Rücklaufgas R enthält vor allem Kohlendioxid, sowie Wasserdampf und in der Synthese-Stufe 13 nicht reagiertes Kohlenmonoxid und Wasserstoff. Um den Prozess steuern zu können, kann zusätzlicher Kohlenstoff mit hohem Heizwert in den Reaktor 31 eingebracht werden, beispielsweise in Form von Kohle oder Schweröl. Diese Zuschlagstoffe A2 können bereits vorgängig dem Ausgangsmaterial A1 zugeschlagen werden, oder separat in den Reaktor 31 eingebracht werden. Die Vermengung von viskosen Substanzen A2 mit festem Ausgangsmaterial A1 erleichtert die Förderung innerhalb des Reaktors. Flüssige Zuschlagstoffe A2 erhöhen auch die Menge an Pyrolysegas, und damit den Betriebsdruck.

Bei der Pyrolyse der ersten Stufe 1 entsteht Pyrolysekoks D, der im Wesentlichen aus festem Kohlenstoff und anorganischen Reststoffen besteht. welcher am Ende des Druckreaktors 31 abgeführt wird. Die bei der Pyrolyse entstehenden Pyrolysegase E beinhalten sowohl gasförmige, als auch bei Raumtemperatur flüssige und feste Substanzen. Die Zusammensetzung der Pyrolysegase E hängt naturgemäss stark von den Ausgangsmaterialien ab, und enthält gegebenenfalls auch Schadstoffe.

Der Pyrolysekoks D wird unter Druck in den Druckreaktor 32 des zweiten Anlagenteils 42 (zweite Stufe 12) gefördert. Geeignet ist wiederum beispielsweise eine geschlossene Förderchnecke. Die Pyrolysegase E werden über eine separate Transportleitung ebenfalls in den zweiten Druckreaktor 32 gefördert. Ein in der Transportleitung angeordneter Verdichter 21.1 fördert die Pyrolysegase in den unter einem höheren Betriebsdruck stehenden zweiten Druckreaktor 32.

Im zweiten Anlagenteil 42, in welchem die zweite Stufe 12 des Verfahrens durchgeführt wird, liegt die Betriebstemperatur zwischen 600 und 1600 °C. In dieser zweiten Stufe 12 erfolgt nun die Vergasung des festen Kohlenstoffs im Pyrolysekoks D mit Kohlendioxid und gegebenenfalls Sauerstoff und/oder Wasserdampf als Vergasungsmittel zu Kohlenmonoxid und Wasserstoff, gemäss den Reaktionen I, II und III.

Das Kohlendioxid stammt primär aus dem Rücklaufgas R. Es kann auch zusätzliches Kohlendioxid T in den Kreislauf eingespeist werden. Der Wasserdampf besteht primär aus der Restfeuchte des Ausgangsmaterials A1. Es kann auch Prozessdampf B eingespeist werden.

Die für den Ablauf dieser endothermen Reaktionen notwendige thermische Energie stammt beispielsweise aus einer partiellen Oxidation des festen Kohlenstoffs (Reaktion III) mit in den zweiten Druckreaktor 32 geleitetem Sauerstoff F. Zum Aufstarten der erfindungsgemässen Anlage und zur Steuerung des Prozesses kann es notwendig sein, dem zweiten Reaktor 32 zusätzliche Brennstoffe G zuzuführen, wie beispielsweise Koks, Öl oder Erdgas, und/oder die Sauerstoffzufuhr, um damit die Wärmeproduktion vorübergehend zu erhöhen.

Das für die spätere Synthese in der dritten Stufe 13 wichtige Verhältnis zwischen Kohlenmonoxid und Wasserstoff ist durch die Wassergas-Shift-Reaktion IV gegeben, und kann durch Zugabe von Prozessdampf B auf die rechte Seite hin beeinflusst werden, Es ist jedoch vorteilhaft, die Gesamtmenge an Wasser im System möglichst gering zu halten, und stattdessen zusätzlichen Wasserstoff direkt in die dritte Stufe einzuleiten.

Im gezeigten Beispiel einer erfindungsgemässen Anlage 1 umfasst die zweite Stufe 12 ebenfalls einen horizontalen Druckkörper 321, in welchem die Förderung des Pyrolysekoks innerhalb des Reaktors 32 durch einen Vorschubrost 322 erfolgt. Wiederum sind auch andere Fördersysteme möglich, wie sie auch bereits für den ersten Druckreaktor 31 besprochen worden sind. Dies hat den Vorteil, dass der Pyrolysekoks ohne weitere Vorbereitung in der zweiten Stufe 12 prozessiert werden kann. Die reaktive Oberfläche des stückigen Pyrolysekoks ist im Vergleich einer ebenfalls möglichen Reaktion im Wirbelstrom vergleichsweise klein, was aber durch die aufgrund der hohen Massenkapazität des Druckreaktors vergleichsweise lange Verweildauer im Reaktor 32 ausgeglichen wird.

Grundsätzlich kann der zweite Reaktor auch anders ausgestaltet sein. Beispielsweise könnte der Pyrolysekoks vorangehend zerkleinert oder gemahlen werden, was dann eine Vergasung des Koks im Wirbelstrom oder Flugstrom ermöglichen würde. Aufgrund der grösseren Oberfläche erlauben solche Reaktoren schnellere Reaktionsgeschwindigkeiten, was jedoch gleichzeitig mit geringeren Massenkapazitäten einhergeht. Zudem erfordern solche Anlagen eine präzisere Steuerung der Gasstromgeschwindigkeit.

Der für die partielle Verbrennung notwendige Sauerstoff F und gegebenenfalls der Prozessdampf B wird in das durch den Pyrolysekoks gebildete Glutbett eingeblasen, wodurch die notwendige thermische Energie erzeugt und der Reaktor 32 auf Betriebstemperatur gehalten wird. Anstatt reinem Sauerstoff könnte auch Luft erwendet werden, wobei jedoch der Luftstickstoff den innerhalb der erfindungsgemässen Anlage kreisenden Gasmateriestrom aufbläht. Dies reduziert die Effizienz der Anlage wesentlich, so dass reiner Sauerstoff in jedem Fall vorzuziehen ist. Zudem verhindert die Abwesenheit von Stickstoff im System auch die Bildung von Stickoxiden.

Die Pyrolysegase E werden im in Figur 1 gezeigten Ausführungsbeispiel einer erfindungsgemässen Anlage in die Gasphase oberhalb des Glutbetts im Druckreaktor 32 eingeblasen, wo die in den Pyrolysegasen E enthaltenen mehratomigen Moleküle bei den herrschenden hohen Temperaturen sehr schnell gecrackt und zerlegt werden. Das in der zweiten Stufe gebildete Synthesegas S enthält darum im wesentlichen keine organischen Moleküle mehr, und kann für die Fischer-Tropsch-Synthese in der dritten Stufe verwendet werden kann. Auch Schadstoffe wie zum Beispiel Dioxin werden zerlegt. Die Sauerstoffzufuhr F ins Glutbett und der Eintrittsort der Pyrolysegase E in den Druckreaktor wird so gewählt, dass keine Bildung von Dioxinen erfolgen kann. Ebenso sollte im austretenden Synthesegas kein Sauerstoff vorhanden sein.

Für unproblematische Ausgangsstoffe, wie beispielsweise Holzschnitzel oder Stroh oder andere unbelastete Biomassen ist es auch möglich, die Pyrolysegase E vorgängig mit Sauerstoff in einem separaten Brenner zu verbrennen, und die heissen Abgase zwecks Zufuhr thermischer Energie ebenfalls ins Glutbett zu leiten, oder unverbrannt direkt ins Glutbett einzublasen, wo sie ebenfalls oxidiert werden.

Am Ende des Druckreaktors 32 verbleiben Reststoffe in Form von Asche H und inerten Reststoffen sowie gegebenenfalls nicht prozessiertem Kohlenstoff. Vorzugsweise sind die Temperaturen so gewählt, dass die Asche verschlackt, so dass die resultierende Schlacke 35 später unproblematisch deponiert werden kann. Es können auch Zuschlagstoffe beigegeben werden, welche den Ascheschmelzpunkt erniedrigen. Zu diesem Zweck kann beispielsweise dem Ausgangsmaterial A1 Kalkpulver zugeschlagen werden. Die Schlacke wird aus dem zweiten Druckreaktor 32 über eine geeignete Druckschleuse 24.2 aus dem Druckbereich der Anlage 1 abgeführt.

Der Synthesegas-Strom S wird aus dem zweiten Druckreaktor 32 abgeführt. Ein Hauptteil S1 wird einem Zyklon 22 zugeführt, wo Staub K, hauptsächlich bestehend aus Restkoks und Asche, abgetrennt wird. Der Reststaub K kann in den ersten 31 oder zweiten 32 Druckreaktor zurückgeführt werden, oder er wird aufbereitet und/oder entsorgt. Anstelle eines Zyklons kann auch eine andere geeignete Gasstromreinigungsvorrichtung verwendet werden.

Anschliessend an die Entfernung des Reststaubs K wird der Synthesgas-Strom durch einen geeigneten Wärmetauscher 23 geleitet, wo der Gasstrom unter Erzeugung von Prozessdampf B auf eine Temperatur abgekühlt wird, die für die Fischer-Tropsch-Synthese in der dritten Stufe 13 geeignet ist. Aufgrund der tieferen Temperaturen sinkt der Druck und verschiebt sich das Gleichgewicht der Reaktionen I, II und IV, wodurch der Anteil an Kohlendioxid im Synthesegas wieder steigt. Ebenfalls kann fester Kohlenstoff L in Form von Graphit aus dem Gasstrom abscheiden. Der Kohlenstoff L kann als Ausgangsmaterial A2 zurück in den Kreislauf geführt werden, oder als Wertstoff anderweitig verwertet werden. Falls der Kohlenstoff L nicht ausgeschieden wird, gelangt er mit dem Synthesegasstrom in dem Fischer-Tropsch-Reaktor 33, wo er zusammen mit dem in der Fischer-Tropsch-Reaktion als Nebenprodukt entstehenden Kohlenstoff ausgeschieden bzw. abfiltriert werden kann. Je nach Ausgangsmaterial kann eine Gastromaufbereitung vorgesehen sein, um störende Stoffe im Synthesegas zu entfernen.

Das Synthesegas S wird nun über eine Druckregulierung 27 einem dritten Druckreaktor 33 des dritten Anlageteils 43 zugeführt, in welchem in einer dritten Stufe 13 eine Fischer-Tropsch-Synthese durchgeführt wird. Die Druckregulierung 27 reduziert den Druck auf den für die dritte Stufe gewünschten Wert. Zur Einstellung des gewünschten Verhältnisses Kohlenmonoxid/Wasserstoff kann zusätzlicher Wasserstoff N in den Fischer-Tropsch-Reaktor 33 geleitet werden. Ebenfalls werden die notwendigen Festkörper-Katalysatoren P zugeführt.

In der Fischer-Tropsch-Synthese der dritten Stufe 13 reagieren das Kohlenmonoxid und der Wasserstoff stark exotherm (ca. 158 kJ/mol pro Kohlenwasserstoffkettenglied bei 250 °C) an heterogenen Katalysatoren (beispielsweise Eisen-, Kobalt-, Ruthenium-, Nickelkatalysatoren) zu Alkanen, Olefinen, Alkoholen, Aldehyden und anderen Kohlenwasserstoffverbindungen und Derivaten. Nebenprodukte sind Methan und fester Kohlenstoff, die ebenfalls in stark exothermen Reaktionen entstehen. Die genauen Parameter der Fischer-Tropsch-Synthese, insbesondere Druck und Temperatur, hängen primär von den herzustellenden Produkten ab, und sind für das grundlegende Funktionsprinzip einer erfindungsgemässen Anlage bzw. das erfindungsgemässe Verfahren nicht direkt relevant. Tendenziell führen höhere Prozesstemperaturen zu kürzeren Kettenlängen und vermehrter Kohlenstoffabscheidung, während höhere Drücke zu längere Kettenlängen führen. Daneben haben die vor allem vorliegenden Partialdrücke von Kohlenmonoxid, Wasserstoff und Wasser einen starken Einfluss auf die Syntheseprodukte.

Für das erfindungsgemässe Verfahren eignen sich beispielsweise Tieftemperatur-Fischer-Tropsch-Verfahren, die bei beispielsweise bei 210 bis 250 °C betrieben werden, und vor allem dieselähnliche Produkte und langkettige Anteile in Form von Wachsen liefern. Letztere können dann zum Beispiel durch Hydrocracking weiter verwertet werden. Hochtemperaturverfahren mit Temperaturen zwischen 320 bis 350 °C wiederum liefern erhebliche Anteile an Methan, kurzkettigen Alkanen und Alkenen, sowie höhere Anteile an Leichtbenzin.

Für Tieftemperaturverfahren eignen sich beispielsweise Rohrbündelreaktoren, in welchen das Synthesegas durch mit Katalysator befüllte, gekühlte Rohre von oben nach unten strömt. Rücklaufgas und Produkte verlassen das Rohr bodenseitig. Besonders geeignet sind moderne Suspensationsreaktoren (schematisch dargestellt in Figur 1), in welchen der feste Katalysator fein verteilt im flüssigen Produkt schwimmt (Slurry). Flüssige Reaktionsprodukte werden aus der flüssigen Phase abfiltriert, während gasförmige Produkte den Reaktor als Teil des Rücklaufgases R verlassen. Die Wärmeabfuhr erfolgt über eingehängte Kühlrohre 331, unter Erzeugung von Prozessdampf C.

Suspensationsreaktoren weisen eine einfachere Bauweise auf als Rohrbündelreaktoren, und sind darum kostengünstig. Der Katalysator kann effizienter genutzt werden, und ist während des laufenden Betriebs austauschbar, was beim erfindungsgemässen zyklischen Verfahren vorteilhaft ist.

Der durch die Kühlvorrichtung 331 gewonnene Prozessdampf C beinhaltet eine erhebliche thermische Energie, ist jedoch nicht heiss genug für eine effiziente Verwertung in einer Dampfturbine oder zur Verwendung für Heizwecke. Er wird deshalb vorteilhaft für die Prozessdampfgewinnung zum Beispiel im Wärmetauscher 23 verwendet, um die allgemeine Energieeffizient der Anlage zu erhöhen und damit die Kohlendioxid-Bilanz weiter zu verbessern.

Der Rücklaufgasstrom R, welcher den Fischer-Tropsch-Reaktor 33 verlässt, enthält neben unreagiertem Kohlenmonoxid und Wasserstoffgas noch Wasserdampf, Kohlendioxid, und gasförmige Reaktionsprodukte. Ein Anteil an leichtflüchtigen Kohlenwasserstoffen kann daraus beispielsweise mit Hilfe einer Kühlkolonne (nicht dargestellt) auskondensiert werden. Ebenso kann Wasser auskondensiert und aus dem Rücklaufgas entfernt werden. Ein Teil R3 des Rücklaufgases kann auch in den dritten Reaktor zurückgeleitet werden, wodurch verbliebene Synthesegasanteile nachreagiert und die Ausbeute erhöht werden kann. Aus dem verbliebenen Rücklaufgasstrom kann ein Teil R2 als Verfahrensprodukt abgetrennt werden. Der verbleibende Rücklaufgasstrom R1 wird in einem Verdichter 21.2 verdichtet und wieder zurück in den ersten Reaktor 31 geführt.

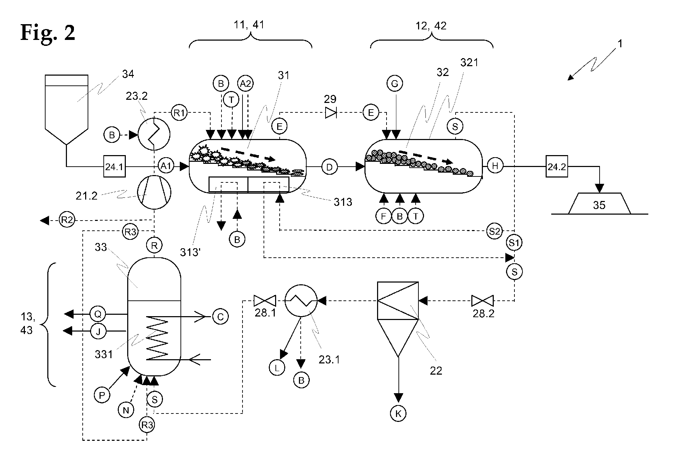

Die zyklische Förderung des Gasstroms innerhalb einer erfindungsgemässen Anlage erfolgt vor allem aufgrund der herrschenden Druckdifferentiale entlang des Kreislaufs. Diese werden primär durch die zwei Verdichter 21.1, 21.2 erzeugt. Je nach Auslegung der Anlage kann auf einen der beiden Verdichter verzichtet werden, was die Gesamtkosten der Anlage senkt. Beinhaltet die Anlage nur einen Verdichter (wie beispielsweise im nachfolgend besprochenen zweiten Ausführungsbeispiel in Figur 2), so hat die Anordnung vor dem ersten Reaktor 31 den Vorteil, dass der entsprechende Verdichter 21.2 ein geringeres Gasvolumen verdichten muss, als ein Verdichter 21.1 zwischen der ersten 11 und zweiten 12 Stufe, wo zusätzlich die Pyrolysegase hinzukommen und das Gesamtvolumen aufgrund der höheren Temperatur grösser ist, oder gar zwischen der zweiten 12 und dritten Stufe 13.

Wird auf den Verdichter 21.1 verzichtet, so besteht zwischen den beiden Reaktoren 31, 32 nur ein geringes Druckgefälle, so dass die erste und zweite Stufe 11, 12 im wesentlichen bei gleichem Druck ablaufen. Der Gasstrom läuft dann also vom Verdichter 21.2 über den ersten 31, zweiten 32 und dritten Reaktor 33 zurück zum Verdichter 21.2. Wird hingegen auf den Verdichter 21.2 verzichtet, ist der Druck innerhalb des dritten und ersten Reaktors 33, 31 im Wesentlichen gleich. Es kann auch ein Verdichter zwischen der zweiten und der dritten Stufe angeordnet werden. Aus Gründen der Entropie muss mindestens ein Verdichter oder sonst ein Fördermittel vorhanden sein, um den Gasstrom zu fördern und das Verfahren am Laufen zu halten.

Zum Ausgleich von vorübergehenden Schwankungen in der Gasproduktion aufgrund von heterogenem Ausgangsmaterial können entlang des Gaskreislaufs E, S, R Druckspeicher vorgesehen sein.

Falls die Anlage aus Figur 1 klein ausgeführt wird, und entsprechend der Volumenstrom E zwischen dem ersten 31 und dem zweiten 32 Druckreaktor vergleichsweise gering ist, kann der Verdichter 21.1 mit vertretbarem Energieaufwand einen Druckunterschied von mehreren Bar erzeugen. Die erste Stufe 11 könnte dann bei einem wesentlich niedrigeren Druck als die zweite Stufe 12 gefahren werden. Die erste Stufe 11 kann sogar bei Normaldruck oder sogar Unterdruck durchgeführt werden.

### Zweites Ausführungsbeispiel

Eine weitere Ausführungsform einer erfindungsgemässen Anlage ist in Figur 2 dargestellt. Im Gegensatz zu Figur 1 ist zwischen dem ersten Druckreaktor 31 und dem zweiten Druckreaktor 32 kein Verdichter angeordnet, sondern lediglich ein Rückschlagventil 29, auf das jedoch gegebenenfalls auch verzichtet werden kann. Der Gasstrom durch die erfindungsgemässe Anlage wird durch das durch den Verdichter 21.2 erzeugte Druckgefälle durch die Anlage gefördert. Da diese vorteilhafte Variante nur mit einem einzigen Verdichter 21.2 auskommt, der zudem ein geringeres Durchsatzvolumen aufweisen muss, sind die Gesamtkosten der Anlage 1 geringer.

In der gezeigten Variante wird der abgezweigte Synthesegas-Strom S2 nicht direkt zurück in den ersten Reaktor 31 geleitet, sondern stattdessen durch eine Heizvorrichtung 313 des Druckreaktors 31 geführt und anschliessend wieder mit dem Synthesegas S1 vereint. Alternativ oder zusätzlich kann eine weitere Heizvorrichtung 313' vorgesehen sein, welche mit überhitztem Prozessdampf B betrieben wird.

Ein Wärmetauscher 23.2 ist im Rücklaufgasstrom R1 angeordnet, und dient der Erhitzung des Rücklaufgasstromes R1 durch Prozessdampf B. Der Rücklaufgasstrom dient in dieser Ausführungsform damit ebenfalls der Wärmezufuhr in den ersten Druckreaktor 31.

Im gezeigten Beispiel ist vor dem dritten Druckreaktor keine Druckreduzierung vorgesehen. Die Drucksteuerung in der dritten Stufe 13 erfolgt dabei direkt über die Drucksteuerung in der zweiten Stufe 12 und den nachfolgenden Druckabfall aufgrund der Abkühlung des Synthesegasstroms S im Wärmetauscher 23.1, sowie den Verdichter 21.2.

### Steuerung und Optimierung der Betriebsparameter

Das in den Figuren 1 und 2 dargestellte erfindungsgemässe Verfahren beruht auf einem zyklischen Stofffluss durch die drei Stufen 11, 12, 13 der Anlage 1, wobei kohlenstoffhaltiges Ausgangsmaterial A1 als Kohlenstofflieferant und Energieträger in den Kreislauf eingespeist und die Produkte Q der Fischer-Tropsch-Stufe 13 als Endprodukt des Verfahrens abgezweigt werden. Die Schlacke H als Restmaterial, sowie Wasserdampf im Rücklaufgas R2 werden laufend aus dem Kreislauf entfernt. Der in den Wärmetauschern entstehende Prozessdampf B wird für den Betrieb der Anlage verwendet, und erhöht so die Effizienz und Effektivität der Anlage. Gegebenenfalls kann überschüssige Prozessdampf-Energie auch zur Energieproduktion verwendet werden, beispielsweise in einer Dampfturbine.

Im Wesentlichen wird in einem erfindungsgemässen Verfahren aus einem energiereichen aber heterogenen und schwer verwertbaren fest Ausgangsmaterial A1 ein wiederum energiereiches Produkt Q hergestellt, nämlich die verschiedenen Fraktionen der Fischer-Tropsch-Stufe 13. Diese können anschliessend weiter verwertet werden, beispielsweise als flüssige Treibstoffe oder als Edukte für die chemische Industrie. Die für den Betrieb der Anlage notwendige Energie stammt aus der partiellen Verbrennungsreaktion in der zweiten Stufe, wobei ein Überschuss der dort erzeugten chemischen Energie (in Form des Synthesegases) später in der exothermen Fischer-Tropsch-Reaktion der dritten Stufe 13 wieder in thermische Energie umgewandelt wird.

Aufgrund des kreisenden Materiestroms im Verfahren liegt während des Betriebs der Anlage ein dynamisches Gleichgewicht vor. Die notwendigen Werte der verschiedenen Parameter (Druck, Temperatur, chemische Zusammensetzungen, Fördergeschwindigkeiten etc.) in den einzelnen Teilen der Anlage werden unter anderem durch die Art des verwendeten Ausgangsmaterials bestimmt. Um trotz heterogenem Ausgangsmaterial einen konstanten Betriebszustand zu halten, können diverse Betriebsparameter gesteuert werden.

Für die Produktion der Kohlenwasserstoffe und anderen Produkte in der dritten Fischer-Tropsch-Stufe 13 sind der Druck und die Temperatur im dritten Reaktor 33 die ausschlaggebenden Parameter. Der Druck kann kurzfristig mit dem Verdichter 21.2 kontrolliert werden, indem dessen Leistung erhöht oder erniedrigt wird. Die Temperatur kann wiederum durch die Kühlleistung des Wärmetauschers 331 gesteuert werden. Langfristig kann der Druck über den Druck im Synthesegasstrom S gesteuert werden, indem zum einen der Betriebsdruck und die Temperatur in der zweiten Stufe 12 geändert wird, und zum anderen die Kühlleistung des Wärmetauschers 23.1 gesteuert wird, und damit der Temperatur- und Druckabfall im Synthesegasstrom S.

Die Steuerung einer erfindungsgemässen Anlage ist vergleichsweise einfach, da die Anlage in einem Gleichgewicht mit Feedback fährt, und zur Steuerung einiger weniger relevanter Parameter eine Mehrzahl von Parametern, die einzelnen Betriebsparameter der verschiedenen Anlagenbestandteile verändert werden können, welche das Gleichgewicht langsam oder schnell beeinflussen können.

Das erfindungsgemässe Verfahren wird vorzugsweise mit einem erhöhten Kohlendioxidanteil durchgeführt. Dies verschiebt unter anderem das Reaktionsgleichgewicht IV nach links (mehr Kohlenmonoxid). Ermöglicht wird ein solcher erhöhter Kohlendioxidgehalt bei gleichzeitig trotzdem möglichst hohen absoluten Mengen an Kohlenmonoxid und damit an Verarbeitungsleistung durch den erhöhten Betriebsdruck der erfindungsgemässen Anlage zwischen 10 und 60 bar. Höhere oder tiefer Drücke sind ebenfalls möglich, aber weniger effizient.

Das erfindungsgemässe Verfahren bzw. die erfindungsgemässe Anlage kann in Bezug auf verschiedene Aspekte optimiert werden. Sollen beispielsweise vor allem aus CO₂-neutraler Biomasse wie zum Beispiel Holzschnitzeln in der dritten Stufe Wertstoffe wie beispielsweise diesel- und benzinanaloge Kohlenwasserstoffe und Wachse etc. erzeugt werden, so wird das Verfahren auf ein möglichst günstiges Verhältnis zwischen Kosten für Biomasse und Betriebskosten und erzeugten Wertsstoffen ausgerichtet. Auf den Ausstoss an Kohlendioxid hingegen muss weniger Rücksicht genommen werden, da es sich ja um CO₂-neutrale Biomasse handelt. Um die ökologische Bilanz weiter zu verbessern, kann die externe Energiezufuhr (elektrischer Strom etc.) reduziert werden, bei gegebenenfalls erhöhtem Biomasseverbrauch.

Liegt hingegen das Schwergewicht auf einer umweltfreundlichen Entsorgung von schadstoffbelasteten Stoffen mit einer minimalen Kohlendioxidproduktion, so wird die Anlage so betrieben, dass möglichst wenig Kohlendioxid aus dem Kreislauf entfernt und and die Umwelt abgegeben werden muss. Dies führt dann gegebenenfalls zu einem erhöhten Bedarf an externer Energie.

Ebenfalls kann die Anlage auf maximalen Durchsatz an Ausgangsmaterial A1 optimiert werden, so dass gegebenenfalls nicht prozessierter Pyrolysekoks die dritte Stufe zusammen mit der Schlacke verlässt. Der umwelttechnisch wenig problematische Pyrolysekoks kann dann zusammen mit der Schlacke deponiert werden.

Die Betriebstemperatur der zweiten Stufe kann ebenfalls optimiert werden. So kann beispielsweise die Temperatur gesenkt werden, um den Mengen-Durchsatz im zweiten Reaktor 32 zu erhöhen. Dies führt dann gegebenenfalls dazu, dass gewisse flüchtige Stoffe im Pyrolysegas E nicht mehr gecrackt werden und mit dem Synthesegas S in den Fischer-Tropsch-Reaktor 33 gelangen. So kann bespielsweise Benzol aus dem Ausgangsmaterial, beispielsweise aus Schweröl, in kleineren Mengen in die Produkte der Fischer-Tropsch-Synthese gelangen. Dort können diese Substanzen entweder verbleiben, beispielsweise als Teil eines flüssigen Treibstoffes, oder falls nötig abgetrennt werden.

### Inbetriebnahme

Nachfolgend wird nun eine Methode erörtert zur Inbetriebnahme einer erfindungsgemässen Anlage wie in Figur 1 dargestellt. Vor Inbetriebnahme wird die Anlage mit einem sauerstofffreien Gas, vorzugsweise Kohlendioxid, gespült und befüllt.

Anschliessend wird der vorgängig mit Koks befühlte zweite Druckreaktor 32 aufgeheizt, beispielsweise mit Gasbrennern. Der zweite Reaktor wird dazu vom Kreislauf abgetrennt, durch Schliessen der entsprechenden Verbindungen. Während des Aufheizens auf die gewünschte Betriebstemperatur wird die Förderung 322 des Koks innerhalb des Druckreaktors 32 noch nicht eingeschaltet. Gegebenenfalls kann im Kreislauf ein temporärer Kurzschluss zwischen Wärmetauscher 23 und Druckreaktor 32 vorgesehen sein (nicht dargestellt), um das erwärmte Gas im System zirkulieren und den gesamten Anlagenabschnitt gleichmässig aufwärmen zu können. Der Druck wird ebenfalls auf den Sollwert erhöht.

Parallel dazu wird der ebenfalls vorgängig mit Koks befüllte erste Druckreaktor 31 vom Kreislauf abgetrennt, und auf die vorgesehene Betriebstemperatur der ersten Stufe aufgeheizt. Der Druck wird ebenfalls auf den gewünschten Wert für die erste Stufe gebracht. Die Materialförderung 312 im ersten Reaktor bleibt noch ausgeschaltet. Das Aufheizen sollte jedoch vorzugsweise ohne Ausgangsmaterial erfolgen, da eine Pyrolyse des Ausgangsmaterials unterhalb einer minimalen sicheren Betriebstemperatur von 450 °C zur Bildung explosiver Gemische führen kann. Der Koks hingegen ist bereits pyrolysiert, und dient lediglich der Zufuhr von Koks in die zweite Stufe, wenn der Kreislauf später in Gang gesetzt wird.

Der Fischer-Tropsch-Reaktor 33 wird ebenfalls vom Kreislauf abgetrennt auf die Betriebsbedingungen hochgefahren. Nach Erreichen der Betriebsbedingungen in den verschiedenen Stufen 11, 12, 13 der Anlage werden die verschiedenen Fördersysteme 312, 322 langsam hochgefahren, der Kreislauf geöffnet, und die Verdichter 21.1, 21.2 in Betrieb gesetzt, so dass schlussendlich ein Gleichgewichtszustand der Anlage bei den gewünschten Betriebsparametern resultiert.

### Drittes Ausführungsbeispiel

In einer weiteren möglichen Variante des erfindungsgemässen Verfahrens wird der Tieftemperatur-Fischer-Tropsch-Reaktor der dritten Stufe 13 durch einen Hochtemperatur-Fischer-Tropsch-Reaktor ersetzt, in welchem der Katalysator als verwirbelter Flugstaub vorhanden ist. Die bei der Hochtemperatur-Fischer-Tropsch-Synthese bevorzugt gebildeten gasförmigen, kurzkettigen Kohlenwasserstoffe, die nach einer ersten Kondensationsstufe im Rücklaufgas verbleiben, werden von den kleineren Molekülen des Rücklaufgases wie Kohlendioxid, Kohlenmonoxid, Wasserstoff durch Permeationsgasfilter abgetrennt. Solche Systeme sind beispielsweise aus der petrochemischen Industrie zur Reinigung von Erdgas bekannt. Im vorliegenden Fall dienen sie dazu, eine erste, kohlenwasserstoffreiche Gasphase und eine zweite, kohlenwasserstoffarme Gasphase zur erzeugen. Die kohlenwasserstoffreiche Gasphase wird weiter verwertet, und beispielsweise zu Flüssiggas und Erdgas verarbeitet. Die kohlenwasserstoffarme und kohlendioxidreiche zweite Gasphase wird als Rücklaufgas zurück in den Kreislauf geschickt.

### Viertes Ausführungsbeispiel

In einer anderen Variante einer erfindungsgemässen Anlage beinhaltet der dritte Anlagenteil 43 anstatt eines Fischer-Tropsch-Reaktors einen Liquid-Phase-Methanol-Synthese-Reaktor. Die aus dem Stand der Technik bekannte Liquid-Phase-Methanol-Synthese ist besonders dazu geeignet, Methanol in hoher Ausbeute aus Synthesegas mit höherem Anteil an Kohlendioxid herzustellen. Die Synthese findet in einem "Slurry-Bubble-Column-Reactor', in welchem das Synthesegas in eine Aufschlämmung des pulverförmigen Katalysators in einem inerten Mineralöl eingeblasen wird. Die Reaktion ist stark exotherm, so dass eine Kühlvorrichtung notwendig ist. Das erzeugte gasförmige Methanol verlässt den Druckreaktor zusammen mit unreagiertem Synthesegas. Nach dem Abscheiden von mitgeschlepptem Mineralöl und Katalysator wird das Methanol auskondensiert.

Methanol ist ein wertvolles Grundprodukt für die chemische Industrie, und kann auch als Treibstoff verwendet werden. Methanol kann zudem als Zuschlagstoff zu Benzin dienen, wobei beispielsweise in Deutschland ein Anteil von bis zu 3% Methanol in Fahrzeugbenzin erlaubt ist.

### Fünftes Ausführungsbeispiel

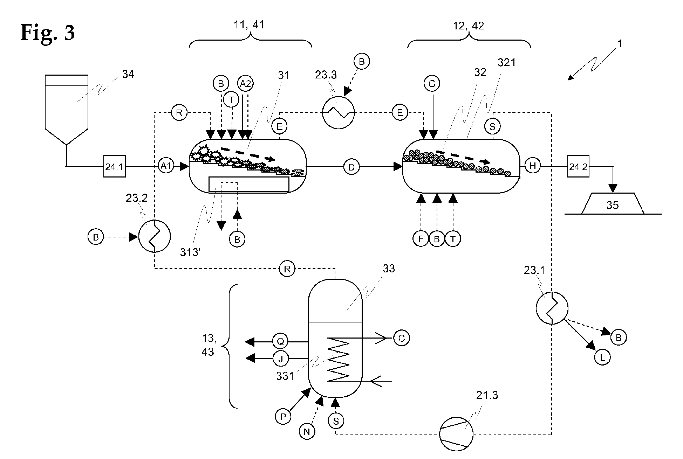

Figur 3 zeigt schematisch noch eine vorteilhafte Ausführungsform einer erfindungsgemässen Anlage 1. Zwischen dem ersten Anlagenteil 41 und dem zweiten Anlagenteil 42 ist ein Wärmetauscher 23.3 angeordnet, welcher dazu dient, die Pyrolysegase E vor dem Eintritt in den zweiten Druckreaktor 32 mit Prozessdampf auf die Betriebstemperatur der zweiten Stufe 12 zu erhitzen. Der Verdichter 21.3 ist in der Transportleitung des Synthesegases S angeordnet, nach einem Wärmetauscher 23.1. Obwohl der Massestrom an dieser Stelle der Anlage am grössten ist, ist aufgrund der stark abgesenkten Temperatur nach dem Wärmetauscher 23.1 das für den Verdichter 21.3 zu bewältigende Gasvolumen nicht allzu gross, und die Betriebtemperatur für den Verdichter günstig.

In der dargestellten Anlage ist kein Zyklon zur Abtrennung von festen Bestandteilen K im Synthesegasstrom vorgesehen. Der Reststaub K tritt ungehindert in die dritte Stufe 13 ein, wo er in der flüssigen Phase im Fischer-Tropsch-Reaktor 33 gebunden wird. Da der Reststaub in Kohlenwasserstoffen unlöslich ist, kann er ohne grossen Aufwand als Reststoff J ausfiltriert werden. Der Verzicht auf den Zyklonabscheider senkt die Kosten der erfindungsgemässen Anlage 1.

### Sechstes Ausführungsbeispiel

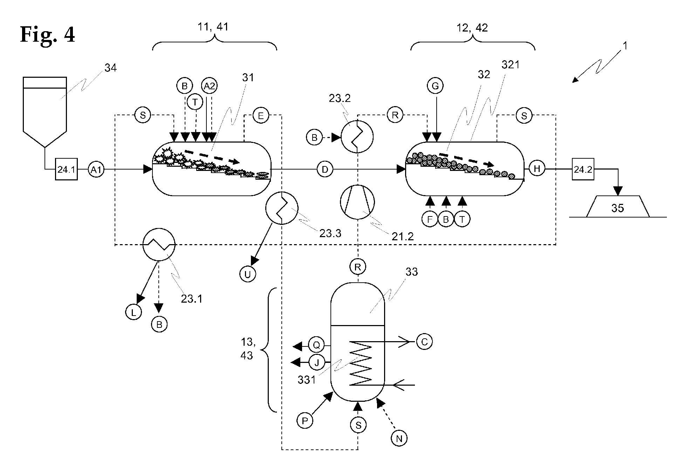

Eine weitere vorteilhafte Ausführungsform einer erfindungsgemässen Anlage ist in Figur 4 dargestellt, welche besonders für die Herstellung von flüssigen Treibstoffen aus unbelasteter Biomasse wie beispielsweise Holzschnitzeln geeignet ist. In dieser Variante werden die Pyrolysegase E nicht in die zweite Stufe 12 sondern in die dritte Stufe 13 geleitet, das Synthesegas S nicht in die dritte Stufe 13 sondern in die erste Stufe 11, und das Rücklaufgas R nicht in die erste Stufe 11 sondern in die zweite Stufe 12.

In der ersten Stufe 11 erhitzt der heisse Synthesegasstrom S das Pyrolysegut und hält die Betriebstemperatur aufrecht. Der aus der ersten Stufe austretende Pyrolysegasstrom E enthält dann neben den eigentlichen Pyrolysegasen auch den Synthesegasanteil aus der zweiten Stufe 12, welcher hier also eine Schleife über die erste Stufe 11 macht.

In der zweiten Stufe wird der Synthesegasanteil in den Pyrolysegasen E in der Fischer-Tropsch-Synthese reagiert, während diejenigen Pyrolysegasanteile, welche nicht bereits im Wärmetauscher 23.3 auskondensiert U sind, sich in der flüssigen Phase des Fischer-Tropsch-Reaktors lösen. Da bei einer direkten Verwendung der Produkte Q der dritten Stufe 13 als Treibstoff die Reinheitsanforderungen gering sind, kann so auf ein Cracken der Pyrolysegase verzichtet werden. Der Treibstoff Q wird anschliessend nachgereinigt, um ungeeignete Reststoffe wie beispielsweise organische Säuren etc. zu entfernen.

Der Rücklaufgasstrom R wird anschliessend verdichtet 21.2, erhitzt 23.2 und in den zweiten Anlagenteil 42 eingeleitet, so dass wieder ein Kreislauf gebildet wird. Da ein Cracken der in den Druckreaktor 32 eingeleiteten Gase nicht notwendig ist, kann die zweite Stufe bei geringerer Betriebstemperatur gefahren werden.

### Siebtes Ausführungsbeispiel

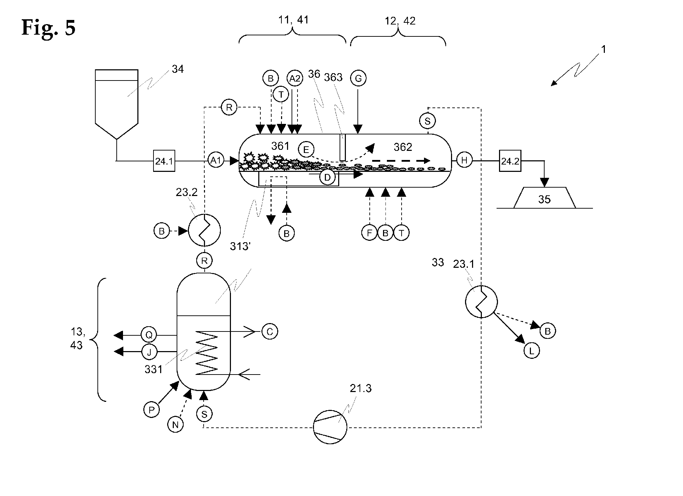

Figur 5 zeigt eine Ausführungsform einer erfindungsgemässen Anlage, beid er die erste Stufe 11 und die zweite Stufe 12 in einem gemeinsamen Druckreaktor 36 durchgeführt werden. Die Pyrolyse findet in einer ersten Kammer 361 statt, die Vergasung in einer zweiten Kammer 362. Die beiden Kammern 361, 362 werden durch eine in Druckreaktor angeordnete Trennwand 363 gebildet, mit einem Durchlass, durch welchen ein gemeinsames Fördersystem den Pyrolysekoks D fördert und das Pyrolysegas E strömt. Die Trennwand 363 dient vor allem dazu, die beiden Kammern 363, 362 thermisch zu isolieren, so dass in den beiden Stufen verschiedene Betriebstemperaturen gefahren werden können.

### Bezugszeichenliste

- 1: Anlage
- 11: erste Stufe
- 12: zweite Stufe
- 13: dritte Stufe
- 21.1, 21.2, 21.3: Verdichter
- 22: Zyklonabscheider
- 23, 23.1, 23.2, 23.3: Wärmetauscher
- 24.1, 24.2: Druckschleuse
- 27: Druckreduzierung
- 28.1, 28.2, 28.3, 28.4: Absperrventil
- 29: Rückschlagventil
- 31: Pyrolyse-Reaktor, erster Druckreaktor
- 31: Druckkörper
- 312: Vorschubrost
- 313, 313': Heizungsvorrichtung
- 32: Vergasungs-Reaktor, zweiter Druckreaktor
- 321: Druckkörper
- 322: Vorschubrost
- 33: Fischer-Tropsch-Reaktor
- 331: Kühlung
- 34: Silo, Vorratsbehälter
- 35: Schlackelager
- 36: gemeinsamer Druckreaktor erste und zweite Stufe
- 361: erste Kammer
- 362: zweite Kammer
- 363: Trennwand
- 41: erster Anlageteil
- 42: zweiter Anlageteil
- 43: dritter Anlageteil
- A1: kohlenstoffhaltiges Ausgangsmaterial
- A2: zusätzliches kohlenstoffhaltiges Ausgangsmaterial (fest, flüssig)
- B: Prozessdampf
- C: Prozessdampf
- D: Pyrolyse-Koks
- E: Pyrolyse-Gas
- F: Sauerstoff, Oxidationsmittel
- G: zusätzliche Brennstoffe
- H: Schlacke, Asche, Reststoffe
- J: Reststoffe
- K: Reststaub
- L: Graphit, Aktivkohle, kohlehaltige Reststoffe
- M: Kühlwasser
- N: Wasserstoffgas
- P: Katalysator
- Q: Produkte der Fischer-Tropsch-Synthese
- R, R1, R2, R3: Rücklaufgas
- S, S1, S2: Synthesegas
- T: Kohlendioxid
- U: schwerflüchtige Anteile des Pyrolysegases

## Patentansprüche

1. Verfahren zur thermisch-chemischen Verwertung von kohlenstoffhaltigen Substanzen (A1), bei welchem in einer ersten Stufe (11) die kohlenstoffhaltigen Substanzen (A1) zugeführt und pyrolysiert werden, wobei Pyrolysekoks (D) und Pyrolysegas (E) entstehen; in einer zweiten Stufe (12) der Pyrolysekoks (D) aus der ersten Stufe (11) vergast wird, wobei Synthesegas (S) entsteht, und Schlacke und andere Reststoffe (H, K) übrig bleiben und abgeführt werden; und in einer dritten Stufe (13) das Synthesegas (S) aus der zweiten Stufe (12) in Kohlenwasserstoffe und/oder andere Produkte (Q) umgewandelt wird, die abgeführt werden; wobei überschüssiges Rücklaufgas (R) aus der dritten Stufe (13) in die erste Stufe (11) und/oder die zweite Stufe (12) geleitet wird, und die drei Stufen (11, 12, 13) einen geschlossenen Kreislauf bilden.

2. Verfahren nach Anspruch 1, **dadurch gekennzeichnet, dass** entlang des Kreislaufs ein Druckgefälle vorliegt.

3. Verfahren nach Anspruch 1 oder 2, **dadurch gekennzeichnet, dass** die Zufuhr der Wärmeenergie für die Pyrolysereaktionen in der ersten Stufe (11) zum Teil oder vollständig durch Rückleiten eines Teils (S2) des heissen Synthesegases aus der zweiten Stufe (12) in die erste Stufe (11) erfolgt, und/oder durch partielle Oxidation des Ausgangsmaterials (A1) und des entstehenden Pyrolysekokses (D).

4. Verfahren nach einem der vorangehenden Ansprüche, **dadurch gekennzeichnet, dass** die erste Stufe (11) bei einer Temperatur zwischen 300 und 800 °C, bevorzugt zwischen 450 und 700 °C, und besonders bevorzugt zwischen 500 und 600 °C, durchgeführt wird.

5. Verfahren nach einem der vorangehenden Ansprüche, **dadurch gekennzeichnet, dass** für die Vergasungsreaktion in der zweiten Stufe (12) Sauerstoff (F) und/oder Wasserdampf (B) und/oder Kohlendioxid (T) als Vergasungsmittel verwendet wird.

6. Verfahren nach einem der vorangehenden Ansprüche, **dadurch gekennzeichnet, dass** in der zweiten Stufe (12) die für die Vergasungsreaktion notwendige thermische Energie zum Teil oder vollständig von aussen zugeführt wird, beispielsweise durch Heizvorrichtungen und/oder Wärmetauscher; und/oder durch Oxidation eines Teils des Pyrolysekokses (D) mit einem Oxidationsmittel, insbesondere Sauerstoff (F), erzeugt wird.

7. Verfahren nach einem der vorangehenden Ansprüche, **dadurch gekennzeichnet, dass** die zweite Stufe (12) bei einer Temperatur zwischen 600 und 1600 °C, bevorzugt zwischen 700 und 1400 °C, und besonders bevorzugt zwischen 850 und 1000 °C, durchgeführt wird.

8. Verfahren nach einem der vorangehenden Ansprüche, **dadurch gekennzeichnet, dass** die erste Stufe (11) und/oder die zweite Stufe (12) bei einem Druck zwischen 10 und 60 bar, vorzugweise zwischen 10 und 25 bar, durchgeführt wird.

9. Verfahren nach einem der vorangehenden Ansprüche, **dadurch gekennzeichnet, dass** die erste Stufe (11) und die zweite Stufe (12) im gleichen Druckreaktor (36) durchgeführt werden.

10. Verfahren nach einem der vorangehenden Ansprüche, **dadurch gekennzeichnet, dass** die Umwandlung in der dritten Stufe (13) mit einer Fischer-Tropsch-Synthese oder einer Liquid-Phase-Methanol-Synthese erfolgt.

11. Anlage (1) zur thermisch-chemischen Verwertung von kohlenstoffhaltigen Substanzen (A1), beinhaltend einen ersten Anlagenteil (41) zur Durchführung einer Pyrolyse der kohlenstoffhaltigen Substanzen (A1) zu Pyrolysekoks (D) und Pyrolysegas (E); einen zweiten Anlagenteil (42) zur Durchführung einer Vergasung des Pyrolysekokses (D) zu Synthesegas (S) und Reststoffen (H, K); einen dritten Anlagenteil (43) zur Durchführung einer Umwandlung des Synthesegases (S) in Kohlenwasserstoffe und/oder andere Produkte (Q); **dadurch gekennzeichnet, dass** alle drei Anlageteile (41, 42, 43) druckfest geschlossen sind und einen im Wesentlichen geschlossenen Kreislauf bilden; eine Transportleitung für das Pyrolysegas (E) den ersten Anlagenteil (41) druckfest mit dem zweiten Anlagenteil (42) und/oder mit dem dritten Anlagenteil (43) verbindet; eine Transportleitung für das Synthesegas (S) den zweiten Anlagenteil (42) druckfest mit dem dritten Anlagenteil (43) und/oder mit dem ersten Anlagenteil (41) verbindet; und eine Transportleitung für das Rücklaufgas (R) den dritten Anlagenteil (43) druckfest mit dem ersten Anlagenteil (41) und/oder mit dem zweiten Anlagenteil (42) verbindet.

12. Anlage nach Anspruch 11, **dadurch gekennzeichnet, dass** entlang mindestens einer der genannten Transportleitungen mindestens ein Verdichter (21.1, 21.2, 21.3) angeordnet ist.

13. Anlage nach einem der Ansprüche 11 oder 12, **dadurch gekennzeichnet, dass** die Anlagenteile (41, 42, 43) je einen oder mehrere Druckreaktoren (31, 32, 33, 36) aufweisen.

14. Anlage nach einem der Ansprüche 11 bis 13, **dadurch gekennzeichnet, dass** der erste Anlagenteil (41) und der zweite Anlagenteil (42) einen gemeinsamen Druckreaktor (36) aufweisen.

15. Kohlenwasserstoffe und andere Produkte (Q), hergestellt mit einem Verfahren nach einem der Ansprüche 1 bis 10 und/oder mit einer Anlage nach einem der Ansprüche 11 bis 14.
